Europäisches Patentamt

(19) European Patent Office

Office européen des brevets

(11) Numéro de publication: **0 270 590**
**B1**

(12) **FASCICULE DE BREVET EUROPEEN**

(45) Date de publication du fascicule du brevet: **18.07.90**

(51) Int. Cl.⁵: **G 01 N 3/32,** G 01 N 33/34

(21) Numéro de dépôt: **87903349.6**

(22) Date de dépôt: **29.05.87**

(86) Numéro de dépôt international:
**PCT/FR87/00188**

(87) Numéro de publication internationale:
**WO 87/07379 03.12.87 Gazette 87/27**

(54) **APPAREIL POUR TESTER LA RESISTANCE AU CLIVAGE DE TUBES DE CARTON.**

(30) Priorité: **29.05.86 FR 8607729**

(43) Date de publication de la demande:
**15.06.88 Bulletin 88/24**

(45) Mention de la délivrance du brevet:
**18.07.90 Bulletin 90/29**

(84) Etats contractants désignés:
**DE GB NL**

(56) Documents cités:
**DE-A-3 021 482**
**DE-A-3 320 971**
**GB-A- 530 418**

(73) Titulaire: **LHOMME SA (Société anonyme)**
**Route de Paris**
**F-89140 Pont sur Yonne (FR)**

(72) Inventeur: **MOREL, Jean-Michel**
**10, rue des Champs-Ribeaux Mallot**
**F-89100 Sens (FR)**

(74) Mandataire: **Chambon, Gérard**
**Cabinet Chambon 6 et 8 avenue Salvador**
**Allende**
**F-93804 Epinay-sur-Seine Cédex (FR)**

EP 0 270 590 B1

Courier Press, Leamington Spa, England.

**Description**

L'invention concerne un appareil pour tester la résistance au clivage de tubes en carton, notamment pour les tubes destinés à servir de mandrins de bobinage utilisés, par exemple, dans l'industrie de l'imprimerie.

Il est connu de bobiner des feuilles de papier sur des mandrins constitués par des tubes en carton. De telles bobines sont manutentionnées par des appareils adaptés pour saisir par l'intérieur les extrémités des tubes servant de mandrins de bobinage.

De même, les bobines, notamment dans l'industrie de l'imprimerie, sont disposées sur des machines de débobinage ou des rotatives en vue de dérouler les bobines pour l'impression de la feuille de papier et/ou son découpage et/ou son pliage, etc. A cet effet, les machines utilisées pour maintenir les bobines comportent deux bras qui viennent s'introduire dans les deux extrémités du tube en carton servant de mandrin de bobinage et qui calent ce dernier au moyen de têtes d'expansion munies de mors capables de faire saillie vers l'extérieur. Les têtes d'expansion sus-mentionnées sont montées libres en rotation, de telle sorte que la bobine peut tourner librement au fur et à mesure de son débobinage.

Il est clair que les mandrins de bobinage sont soumis à de très fortes contraintes, dues au poids de la bobine, à la vitesse et la durée de son débobinage, à la pression exercée par les têtes d'expansion et, parfois, aux contraintes de freinage des têtes d'expansion qui se bloquent en rotation, en cas, par exemple, d'arrêt d'urgence alors que la bobine présente une énergie cinétique importante.

Les caractéristiques des tubes de carton choisis pour servir de mandrin de bobinage sont donc importantes et, jusqu'à présent, la matière utilisée, l'épaisseur des tubes et les autres paramètres de fabrication sont choisis de manière empirique avec pour seul test de résistance, l'essai réel en fonctionnement, en partant de la constatation empirique qu'un tube ayant résisté au-delà d'un temps donné, en fonction du poids de la bobine, il devait résister tout le temps de son utilisation.

Il a été constaté que les tubes carton servant de mandrins de bobinage se détruisent par clivage ou délaminage de la matière au bout d'une durée qui est fonction des contraintes mentionnées ci-avant et plus paticulièrement en fonction du poids de la bobine. On peut en effet observer qu'au fur et à mesure de la rotation de la bobine (généralement très rapide), chaque zone du mandrin, qui tourne avec ladite bobine, est soumise alternativement à des efforts très différents, l'écrasement dû au poids s'effectuant seulement lorsque ladite zone est tournée vers le haut.

Il existe déjà des appareils pour tester la résistance des tubes à la compression, au fléchage, à la dureté (par billage par example). Ces essais sont utiles mais très insuffisants lorsqu'il s'agit de déterminer les caractéristiques d'un tube destiné à servir notamment, de mandrin de bobinage.

C'est pourquoi l'inventeur a imaginé un appareil destiné à tester la résistance au clivage des tubes en carton qui sont, à cet effet, découpés sous forme d'échantillons ou d'éprouvettes.

L'appareil selon l'invention est caractérisé en ce qu'il comporte un mandrin de calage qui est monté libre en rotation, au moins un manchon pour chaque diamètre extérieur différent des éprouvettes et dont le diamètre intérieur est voisin et au moins égal audit diamètre extérieur des éprouvettes, de manière que pour chaque éprouvette, corresponde au moins un manchon destiné à loger ladite éprouvette, le mandrin de calage étant adapté pour recevoir, en la calant, chaque éprouvette entourée d'un manchon correspondant, tandis qu'une courroie d'entaînement est prévue pour être tendue entre ledit manchon et un dispositif d'entraînement, afin de pouvoir entraîner en rotation l'ensemble constitué par le mandrin de calage, une éprouvette et un manchon.

L'appareil selon l'invention reconstitue les contraintes auxquelles sont soumis, notamment, les mandrins de bobinage, les manchons représentant matériellement les bobines dont le poids est simulé par la tension de la courroie d'entraînement.

Selon un mode de réalisation le diamètre intérieur d'un manchon est très légèrement supérieur au diamètre extérieur des éprouvettes correspondantes.

Généralement, ce jeu sera toujours le même quel que socit le diamètre intérieur de l'éprouvette à tester et sera, par exemple, d'environ 2/10 de mm, ce qui n'empêche pas une introduction à force des éprouvettes dans lesdits manchons.

Selon un autre mode de réalisation, plusieurs manchons sont prévus dont les diamètres extérieurs sont tous identiques quels que soient leurs diamètres intérieurs.

De cette façon, il est possible de tester plusieurs éprouvettes ayant des diamètres extérieurs différents, alors que la vitesse d'entraînement pourra être constante, étant donné le diamètre extérieur constant des manchons.

Selon un mode de réalisation, le mandrin de calage est un mandrin à mors expansibles vers l'extérieur afin de caler sous une certaine pression chaque éprouvette à tester.

De préférence dans ce cas, la mandrin de calage est muni d'un moyen de réglage et de mesure de la pression exercée par ses mors sur les éprouvettes.

Afin de simuler des poids différents de bobines, la courroie d'entraînement peut être munie d'un moyen de tension réglable.

Le mandrin de calage peut être muni d'un dispositif de freinage et le dispositif d'entraînement de la courroie d'entraînement d'un système de débrayage, tandis qu'un volant d'inertie agissant sur ladite courroie d'entraînement est prévu, afin de tester la résistance des éprouvettes au freinage.

Selon un autre mode de réalisation, le dispositif d'entraînement comporte un moteur muni d'un arbre d'entraînement relié par une courroie de transmission à une poulie de transmission qui est montée libre en rotation mais qui est solidaire en rotation avec une poulie d'entraînement, elle-même en prise avec la courroie d'entraînement.

Les poulies de transmission et d'entraînement sont calées sur un axe de rotation commun, ce dernier et l'axe du mandrin de calage étant parallèles et disposés dans un plan sensiblement horizontal, tandis que l'arbre du moteur d'entraînement est disposé sensiblement à l'aplomb de l'axe de rotation des poulies.

Selon encore un autre mode de réalisation le moteur est fixé sur une plaque support montée pivotante sur un bâti, tandis que l'axe de rotation des poulies de transmission et d'entraînement est monté dans des paliers qui sont fixés sur ladite plaque support, laquelle est reliée de manière articulée à un vérin, destiné à agir sur ladite plaque et donc sur l'axe de rotation, afin de régler la tension de la courroie d'entraînement, en modifiant l'entraxe compris entre ledit axe de rotation et l'axe du mandrin de calage.

L'invention sera bien comprise à la lecture de la description qui va suivre et qui se réfère aux dessins annexés, dans lesquels:

la figure 1 est une élévation schématique d'un appareil selon l'invention,

la figure 2 est une vue en plan de dessus de la figure 1, partiellement en coupe,

la figure 3 est une vue agrandie, partiellement en coupe, de l'extrémité du mandrin de calage supportant une éprouvette et un manchon,

la figure 4 est une coupe selon IV—IV de la figure 3.

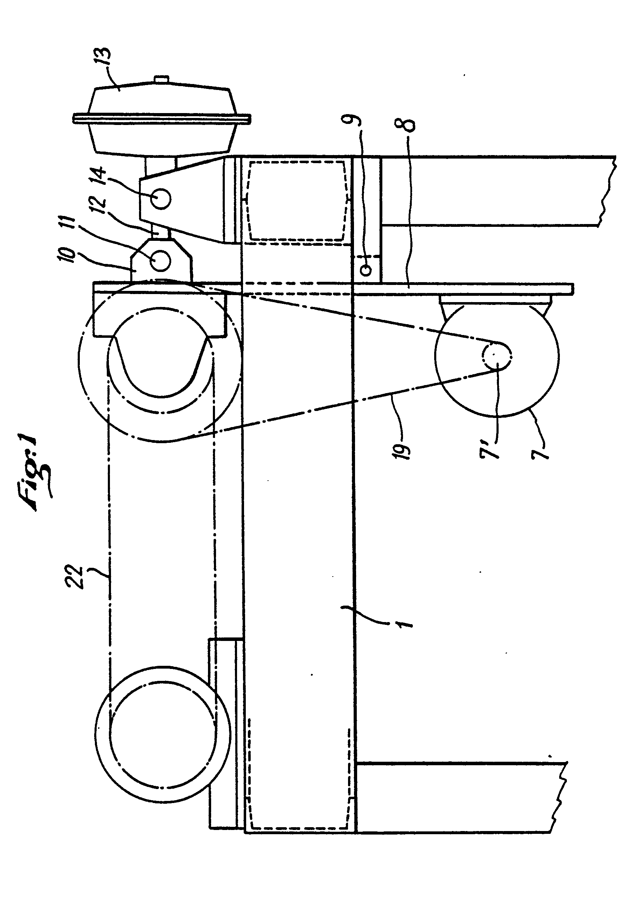

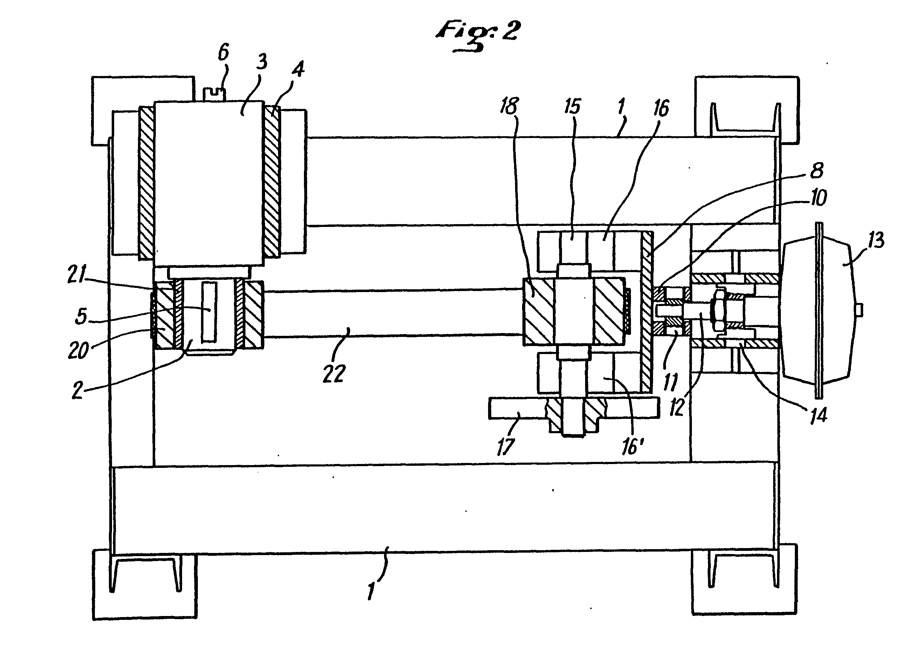

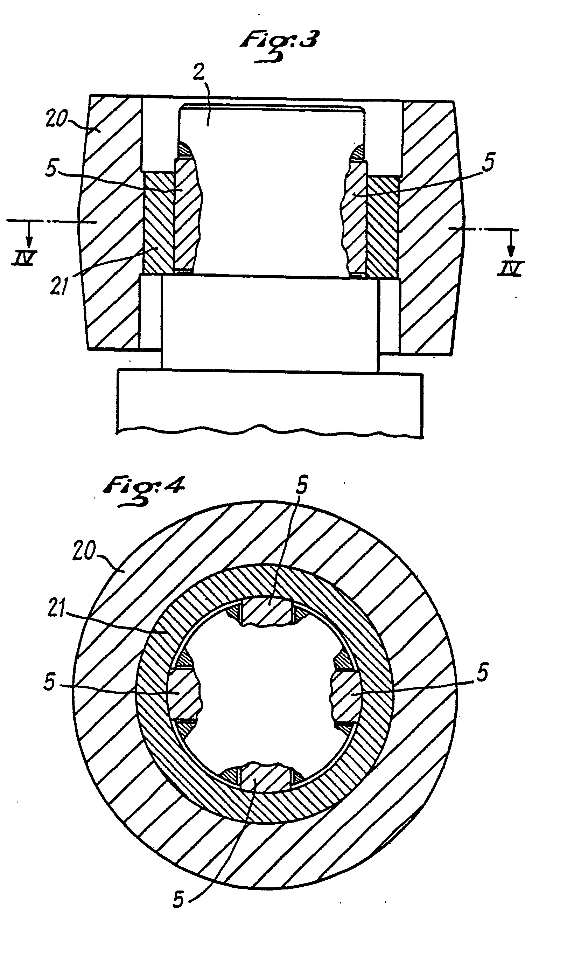

L'appareil selon l'invention est fixé sur un bâti 1 (figures 1 et 2) et comporte un mandrin 2 (figures 2 à 4) dont l'une des extrémités est montée libre en rotation dans un palier 3, fixé au bâti 1 au moyen d'un support annulaire 4 (figure 2).

Le mandrin 2 est muni, à son extrémité libre, de mors 5 (figures 2 à 4) expansibles vers l'extérieur par tout moyen connu, aménagé dans le palier 3, et manoeuvrable, par exemple, par un écrou ou une vis 6 (figure 2) qui permet en outre un réglage de la pression de serrage des mors 5, comme il sera précisé ci-après.

Un moteur d'entraînement 7 muni d'un arbre rotatif 7' de sortie (figure 1) est fixé sur une plaque support 8, sensiblement verticale qui est articulée, d'une part, de manière pivotante autour de l'axe 9 au bâti 1 (figure 1) et, d'autre part, au moyen d'un étrier 10 et d'un axe 11 à la tige 12 d'un vérin pneumatique 13 (figures 1 et 2). Le cylindre du vérin 13 est en outre articulé de manière pivotante autour d'un axe 14 du bâti 1 (figures 1 et 2).

Sensiblement à l'aplomb et au-dessus de l'arbre moteur 7', est aménagé un axe 15 monté libre en rotation dans des paliers 16 et 16' solidaires de la plaque support 8 (figure 2). L'axe 15 est parallèle à l'arbre 7 et à l'axe géométrique du mandrin 2 avec lequel il définit un plan sensiblement horizontal, tandis que sur ledit axe 15, sont calées deux poulies 17 et 18 (figure 2).

Entre l'arbre moteur 7' et la poulie 17, est tendue une courroie de transmission 19 (figure 1).

Pour tester des tubes en carton, on découpe des tranches destinées à servir d'échantillons ou d'éprouvettes.

L'invention prévoit de disposer d'une pluralité de manchons tels que 20 sur les figures 2, 3 et 4.

Tous les manchons peuvent présenter avantageusement le même diamètre extérieur et divers diamètres intérieurs, en fonction des éprouvettes à tester, comme il sera dit ci-après.

Généralement, le diamètre intérieur des éprouvettes, lorsqu'il s'agit de tester des mandrins de bobinage, est standard, de telle sorte que leurs diamètres extérieurs seront différents du seul fait des épaisseurs différentes des éprouvettes, mais il est clair que le diamètre extérieur des éprouvettes peut être quelconque.

Chaque éprouvette 21 (figures 2, 3 et 4) à tester est introduite dans un manchon 20 correspondant, c'est-à-dire ayant un diamètre intérieur au moins égal au diamètre extérieur de ladite éprouvette. L'inventeur a toutefois trouvé avantageux de prévoir une cote supérieure d'environ 2/10 de mm pour le diamètre intérieur du manchon, dans sa partie destinée à être en contact avec l'éprouvette, par rapport au diamètre extérieur de ladite éprouvette (ce jeu étant de préférence le même pour toutes les éprouvettes et les manchons correspondants). Sur la figure 2, la longueur de l'éprouvette 21 est identique à la longueur du manchon 20. Toutefois, à titre d'exemple, on a représenté sur la figure 3, une éprouvette légèrement plus courte que son manchon, ce qui permet notamment de prévoir éventuellement (comme le montre ladite figure 3) un jeu plus important entre les diamètres sur les entrées du manchon, en facilitant ainsi l'emmanchement de l'éprouvette.

Lorsque l'éprouvette 21 est logée dans son manchon, l'ensemble est disposé sur le mandrin 2 (figures 2, 3 et 4), et on agit sur la vis de réglage 6 pour assurer l'expansion des mors qui exercent alors une certaine pression sur la paroi interne de l'éprouvette.

Une courroie de transmission 22 (figures 1 et 2) est alors tendue entre le manchon 20 et la poulie 18.

Le vérin pneumatique 13 permet de régler la tension de la courroie 22 en basculant plus ou moins le support 8 qui pivote en 9 sur le bâti 1, ce qui modifie l'entraxe entre l'axe géométrique du mandrin 2 et l'axe 15 des poulies 17 et 18.

Le moteur 7, par l'intermédiaire de son arbre 7', de la courroie de transmission 19 et de la poulie 17, permet d'entraîner en rotation la poulie 18 (solidaire de l'axe 15) et ainsi la courroie 22. La courroie 22 entraîne alors en rotation l'ensemble constitué par le mandrin 2, l'éprouvette 21 et le manchon 20.

Le manchon 20 représente matériellement une bobine dont le poids est déterminé par la tension réglable de la courroie 22.

De très nombreux types d'essais peuvent alors être effectués.

Il est en effet possible de faire varier et régler, la pression exercée par les mors 5 (vis 6), la tension de la courroie 22 (vérin 13) et bien sûr aussi la vitesse d'entraînement de ladite courroie, en utilisant un moteur 7 à vitesse variable ou des poulies de divers diamètres pour la courroie 19 et qui sont destinées à être calées sur l'arbre 7' (la position en hauteur du moteur 7 sur la plaque 8 étant alors éventuellement réglable).

Il est par exemple possible de mesurer la durée de vie (jusqu'à la destruction par clivage ou délaminage) d'une éprouvette sous une tension constante de la courroie 22, ou de déterminer une valeur de rupture en faisant varier la tension de la courroie 22 en fonction du temps.

D'autres moyens sont en outre prévus, tels qu'un dispositif de freinage du mandrin 2 pour étudier le comportement de l'éprouvette en cas de freinage brusque (arrêt d'urgence par exemple). Dans ce cas, le moteur 7 est alors muni d'un dispositif de débrayage et l'axe 15 est pourvu d'un volant d'inertie afin d'effectuer des essais de résistance des éprouvettes au freinage apnès avoir débrayé le moteur 7, le volant d'inertie simulant l'inertie d'une bobine en rotation. Il est toutefois possible que la poulie 17 soit choisie de manière convenable pour faire office de volant d'inertie.

Dans le mode de réalisation représenté, la tension de la courroie 22 est en fait fonction de la pression pneumatique du vérin 13, mais il est également prévu un réglage fin de la tension de la courroie au moyen, par exemple, d'un réa de renvoi disposé sur la courroie 22 et qui serait combiné avec un dynamomètre.

L'invention qui concerne plus particulièrement des tests pour déterminer la nature des tubes en carton à choisir pour constituer des mandrins de bobinage, n'est pas forcément limitée à cette application, mais peut également s'appliquer aux essais de tubes soumis à des contraintes voisines ou semblables à celles des mandrins de bobinage.

Enfin, de nombreuses variantes peuvent évidemment être imaginées. La disposition horizontale de la courroie 22 n'est pas obligatoire, le vérin 13 peut être hydraulique ou remplacé par tout autre moyen mécanique, de même que la vis de réglage 6 peut également être remplacée par un autre moyen.

**Revendications**

1. Appareil pour tester la résistance au clivage de tubes en carton, préalablement découpés sous forme d'éprouvettes (21), caractérisé en ce qu'il comporte un mandrin de calage (2) qui est monté libre en rotation, au moins un manchon (20) pour chaque diamètre extérieur différent des éprouvettes (21) et dont le diamètre intérieur est voisin et au moins égal audit diamètre extérieur des éprouvettes (21), de manière que pour chaque éprouvette (21), corresponde au moins un manchon destiné à loger ladite éprouvette (21), le mandrin de calage (2) étant adapté pour recevoir, en la calant, chaque éprouvette (21) entourée d'un manchon correspondant (20), tandis qu'une courroie d'entraînement (22) est prévue pour être tendue entre ledit manchon (20) et un dispositif d'entraînement (7, 7', 19, 17, 18) afin de pouvoir entraîner en rotation l'ensemble constitué par le mandrin de calage (2), une éprouvette (21) et un manchon (20).

2. Appareil selon la revendication 1, caractérisé en ce que le diamètre intérieur d'un manchon (20) est très légèrement supérieur au diamètre extérieur des éprouvettes (21) correspondantes.

3. Appareil selon la revendication 1 ou 2, caractérisé en ce que plusieurs manchons (20) sont prévus dont les diamètres extérieurs sont tous identiques quels que soient leurs diamètres intérieurs.

4. Appareil selon l'une des revendications 1 à 3, caractérisé en ce que le mandrin de calage (2) est un mandrin à mors (5) expansibles vers l'extérieur afin de caler sous une certaine pression chaque éprouvette (21) à tester.

5. Appareil selon la revendication 4, caractérisé en ce que le mandrin de calage (2) est muni d'un moyen (6) de réglage et de mesure de la pression exercée par ses mors (5) sur les éprouvettes (21).

6. Appareil selon l'une des revendications 1 à 5, caractérisé en ce que la courroie d'entraînement (22) est munie d'un moyen de tension réglable (13).

7. Appareil selon l'une des révendications 1 à 6, caractérisé en ce que le mandrin de calage (2) est muni d'un dispositif de freinage et le dispositif d'entraînement de la courroie d'entraînement (22) d'un système de débrayage, tandis qu'un volant d'inertie agissant sur ladite courroie d'entraînement (22) est prévu, afin de tester la résistance des éprouvettes (21) au freinage.

8. Appareil selon l'une des revendications 1 à 7, caractérisé en ce que le dispositif d'entraînement comporte un moteur (7) muni d'un arbre d'entraînement (7') relié par une courroie de transmission (19) à une poulie de transmission (17) qui est montée libre en rotation mais qui est solidaire en rotation avec une poulie d'entraînement (18), elle-même en prise avec la courroie d'entraînement (22).

9. Appareil selon la revendication 8, caractérisé en ce que les poulies de transmission (17) et d'entraînement (18) sont calées sur un axe de rotation commun (15), ce dernier et l'axe du mandrin de calage (2) étant parallèles et disposés dans un plan sensiblement horizontal, tandis que l'arbre (7') du moteur d'entraînement (7) est disposé sensiblement à l'aplomb de l'axe de rotation (15) des poulies (17 et 18).

10. Appareil selon la revendication 9, caractérisé en ce que le moteur (7) est fixé sur une plaque support (8) montée pivotante sur un bâti (1), tandis que l'axe de rotation (15) des poulies de transmission (17) et d'entraînement (18) est monté dans des paliers (16, 16') qui sont fixés sur ladite plaque support (8), laquelle est reliée de manière articulée à un vérin (13), destiné à agir sur ladite plaque (8) et donc sur l'axe de rotation (15), afin de régler la tension de la courroie d'entraînement (22), en modifiant l'entraxe compris entre ledit axe de rotation (15) et l'axe du mandrin de calage (2).

**Patentansprüche**

1. Vorrichtung zum Prüfen des Spaltwiderstandes von Röhren aus Pappe, die vorher in Form von Probestücken (21) unterteilt worden sind, dadurch gekennzeichnet, daß die Vorrichtung einen Haltedorn (2), der frei drehbar angeordnet ist, und wenigstens eine Hülse (20) für jeden unterschiedlichen Außendurchmesser der Probestücke (21) aufweist, wobei der Innendurchmesser der Hülse annähernd und wenigstens gleich dem Außendurchmesser der Probestücke (21) ist, so daß für jedes Probestück (21) wenigstens eine entsprechende Hülse zum Aufnehmen des letzteren vorhanden ist und der Haltedorn zum Aufnehmen von jedem, von der entsprechenden Hülse umgebenen Probestück durch Verkeilen geeignet ist und ein Antriebsriemen (22) um die Hülse (20) und eine Antriebseinheit (7, 7', 17, 18, 19) gespannt ist, um die Anordnung bestehend aus dem Haltedorn (2), einem Probestück (21) und einer Hülse in Drehbewegung zu versetzen.

2. Vorrichtung nach Anspruch 1, dadurch gekennzeichnet, daß der Innendurchmesser einer Hülse (20) sehr wenig größer als der Außendurchmesser der entsprechenden Probestücke (21) ist.

3. Vorrichtung nach Anspruch 1 oder 2, dadurch gekennzeichnet, daß verschiedene Hülsen (20) vorgesehen sind, die alle den gleichen Außerndurchmesser unabhängig von ihren Innendurchmessern aufweisen.

4. Vorrichtung nach einem der Ansprüche 1-3, dadurch gekennzeichnet, daß der Haltedorn ein Dorn mit Backen (5) ist, die nach außen expandierbar sind, um jedes Probestück (21) mit einem bestimmten Druck festzukeilen.

5. Vorrichtung nach Anspruch 4, dadurch gekennzeichnet, daß der Haltedorn (2) mit einer Regel- und Meßeinrichtung (6) für den Druck versehen ist, der von den Backen (5) auf die Probestücke (21) ausgeübt wird.

6. Vorrichtung nach einem der Ansprüche 1-5, dadurch gekennzeichnet, daß der Antriebsriemen (22) mit Mitteln zur Spannungsregulierung (13) versehen ist.

7. Vorrichtung nach einem der Ansprüche 1-6, dadurch gekennzeichnet, daß der Haltedorn (2) mit einer Bremseinrichtung und die Antriebseinrichtung des Antriebsriemens (22) mit einer Auskupplungsvorrichtung versehen sind, wobei ein auf den Antriebsriemen (22) einwirkendes Schwungrad vorgesehen ist, um den Widerstand der Probestücke (21) beim Bremsen zu prüfen.

8. Vorrichtung nach einem der Ansprüche 1-7, dadurch gekennzeichnet, daß die Antriebseinrichtung einen Motor (7) aufweist, der mit einer Antriebswelle (7') versehen ist, die über einen Antriebsriemen (19) mit einer Riemenscheibe (17) verbunden ist, die frei drehbar angeordnet, aber drehfest mit einer Antriebsscheibe (18) verbunden ist, die selbst mit dem Antriebsriemen (22) zusammenwirkt.

9. Vorrichtung nach Anspruch 8, dadurch gekennzeichnet, daß die Riemenscheibe (17) und die Antriebsscheibe (18) auf einer gemeinsamen Rotationsachse (15) befestigt sind, wobei letztere und die Achse des Haltedornes (2) parallel verlaufen und in einer im wesentlichen horizontalen Ebene angeordnet sind, während die Welle (7') des Antriebsmotors (7) im wesentlichen senkrecht zu der Rotationsachse (15) der Scheiben (17) und (18) angeordnet ist.

10. Vorrichtung nach Anspruch 9, dadurch gekennzeichnet, daß der Motor (7) auf einer Stützplatte (8) befestigt ist, die schwenkbar an einem Rahmen (1) angebracht ist und die Rotationsachse der Riemenscheie (17) und der Antriebsscheibe (18) in Lagern (16, 16') angebracht ist, die an der Auflageplatte (8) befestigt sind, welche gelenkig mit einem Zylinder (13) verbunden ist, der dazu bestimmt ist, auf die Stützplatte (8) und somit auf die Rotationsachse (15) einzuwirken, um die Spannung des Antriebsriemens (22) zu regulieren und den Mittenabstand zwischen der Rotationsachse (15) und der Achse des Haltedornes (2) zu verändern.

**Claims**

1. Apparatus for testing the resistance to cleavage of cardboard tubes previously cut in the form of testpieces (21), characterized in that it comprises a holding mandrel (2) which is mounted free to rotate, at least one sleeve (20) for each different outside diameter of the testpieces (21) and the inside diameter of which is close and at least equal to said outside diameter of the testpieces (21) so that for each testpiece (21) there is at least one corresponding sleeve designed to house said testpiece (21), the holding mandrel (2) being adapted to receive and hold each testpiece (21) surrounded by a corresponding sleeve (20), while a driving belt (22) is provided to be stretched between said sleeve (20) and a driving device (7, 7', 19, 17, 18) in order to be able to rotate the assembly constituted by the holding mandrel (2), a testpiece (21) and a sleeve (20).

2. Apparatus as in claim 1, characterized in that the inside diameter of a sleeve (20) is very slightly greater than the outside diameter of the corresponding testpieces (21).

3. Apparatus as in claim 1 or 2, characterized in that a plurality of sleeves (20) are provided the outside diameters of which are all identical irrespective of their inside diameters.

4. Apparatus as in one of claims 1 to 3, characterized in that the holding mandrel (2) is a mandrel with jaws (5) which can be expanded outwards in order to hold each testpiece (21) to be tested with a certain pressure.

5. Apparatus as in claim 4, characterized in that the holding mandrel (2) is provided with a means (6) for adjusting and measuring the pressure exerted by its jaws (5) on the testpieces (21).

6. Apparatus as in one of claims 1 to 5, characterized in that the driving belt (22) is provided with an adjustable tensioning means (13).

7. Apparatus as in one of claims 1 to 6, characterized in that the holding mandrel (2) is provided with a braking device and the driving device of the driving belt (22) with a disengaging system, while an inertia flywheel acting on said driving belt (22) is provided in order to test the resistance of the testpieces (21) to braking.

8. Apparatus as in one of claims 1 to 7, characterized in that the driving device comprises a motor (7) provided with a driving shaft (7′) connected by a drive belt (19) to a drive pulley (17) which is mounted free to rotate but is locked in rotation to a driving pulley (18) which is itself engaged with the driving belt (22).

9. Apparatus as in claim 8, characterized in that the drive pulley (17) and the driving pulley (18) are fixed on a common rotating shaft (15), the latter and the shaft of the holding mandrel (2) being parallel and disposed in a substantially horizontal plane, while the shaft (7′) of the driving motor (7) is disposed substantially plumb with the rotating shaft (15) of the pulleys (17 and 18).

10. Apparatus as in claim 9, characterized in that the motor (7) is fixed on a supporting plate (8) mounted pivotably on a frame (1), while the rotating shaft (15) of the drive pulley (17) and the driving pulley (18) is mounted in bearings (16, 16′) which are fixed on said supporting plate (8) which is articulated to a jack (13) designed to act on said plate (8) and thus on the rotating shaft (15) in order to adjust the tension of the driving belt (22) by modifying the distance between said rotating shaft (15) and the shaft of the holding mandrel (2).

Fig:1

EP 0 270 590 B1

Fig.2

*Fig:3*

*Fig:4*

3